# FASCICULE DE BREVET EUROPEEN

(11) **EP 3 493 881 B1**
(45) Date de publication et mention de la délivrance du brevet: **13.05.2020**
(21) Numéro de dépôt: 17758577.5
(22) Date de dépôt: 18.07.2017
(51) Int. Cl.: A61Q 19/00, A61K 8/81, A61K 8/04, A61K 8/20, A61K 8/49

(54) **SÉRUM COSMÉTIQUE LIMPIDE ÉPAISSI ET RICHE EN SELS, PROCÉDÉ POUR ÉCLAIRCIR UN HYDROGEL ET UTILISATION EN COSMÉTIQUE**
VERDICKTES, KLARES, SALZREICHES KOSMETISCHES SERUM, VERFAHREN ZUR AUFHELLUNG EINES HYDROGELS UND VERWENDUNG DAVON IN DER KOSMETIK
THICKENED, CLEAR SALT-RICH COSMETIC SERUM, METHOD FOR LIGHTENING A HYDROGEL, AND USE THEREOF IN COSMETICS

(30) Priorité: 05.08.2016 FR 1657591
(43) Date de publication de la demande: 12.06.2019
(73) Titulaire: Société d'Exploitation de Produits pour les Industries Chimiques SEPPIC, 75321 Paris Cedex 7 (FR)
(72) Inventeur: VERDU, Benoît, 81100 Castres (FR); SOURDON, Cindy, 81110 Verdalle (FR); ROSO, Alicia, 81710 Saïx (FR)
(74) Mandataire: Laigneau, Amandine
(86) Numéro de dépôt international: PCT/FR2017/051952
(87) Numéro de publication internationale: WO 2018/024961

(56) Documents cités:
- FR-A1- 2 910 899
- US-A1- 2012 172 457
- US-A1- 2014 335 039
- US-A1- 2015 335 567

## Description

La présente invention a pour objet un nouvel hydrogel épaissi et riche en sels, son utilisation en tant que composition cosmétique topique, ainsi qu'un nouveau procédé pour éclaircir un hydrogel épaissi riche en sels.

Les sérums sont largement utilisés aujourd'hui pour le traitement cosmétique de la peau. Par « sérum », on désigne, des solutions aqueuses qui ont pour objectif d'être absorbées rapidement par les couches superficielles de l'épiderme, et qui contiennent une concentration élevée en ingrédients actifs. Ils sont généralement utilisés en complément aux différentes formulations de soin de la peau pour en augmenter l'effet : la peau est préalablement nettoyée, le sérum y est ensuite appliqué puis la formulation de soin cosmétique souhaitée. Ce procédé est particulièrement pertinent lorsque l'utilisateur constate que l'effet de la formulation de soin est diminué, en raison de facteurs externes comme les conditions climatiques ou environnementales, ou en cas de changement brutal de l'état de la peau comme son assèchement. Les sérums comprennent des substances présentant des propriétés diverses, comme des propriétés hydrantes, éclaircissantes, purifiantes, anti-âge, antirides, énergisantes, anticernes et/ou anti-sébum.

Pour que les sérums soient facilement et rapidement absorbés par les couches superficielles de l'épiderme, ils doivent présenter une texture non grasse, présentant un équilibre entre des caractéristiques d'écoulement suffisamment fluides pour être rapidement étalés sur la surface de la peau à traiter et suffisamment gélifiés pour éviter les coulures au moment de l'application. De plus il est préféré que le sérum soit translucide car cet aspect est associé à la notion de pureté par les consommateurs.

Les polymères synthétiques et les polymères d'origines naturelles sont des agents épaississants de phases aqueuses cosmétiques bien connus de l'homme du métier et qui permettent de préparer un gel aqueux pour des utilisations topiques dans le domaine de la cosmétique et de la pharmacie.

Les polymères épaississants synthétiques actuellement utilisés dans ce domaine, se présentent sous forme de poudre ou sous forme liquide en tant qu'une émulsion eau-dans huile du polymère préparé par polymérisation radicalaire en émulsion inverse à l'aide d'agents tensioactifs, couramment nommée latex inverse.

Parmi les polymères épaississants synthétiques en poudre les plus connus, on peut citer les polymères à base d'acide acrylique et/ou de ses esters comme ceux commercialisés sous les noms, Carbopol™ ou Pemulen™. Ils sont décrits notamment dans les brevets américains US 5,373,044, US 2,798,053 ainsi que dans la demande de brevet européen EP 0 301 532 A2. Il y a aussi les polymères à base d'acide 2-acrylamido-2-méthyl propanesulfonique et/ou de ses sels comme ceux commercialisés sous le nom Aristoflex™. Ils sont décrits notamment dans les brevets européens EP 0 816 403, EP 1 116 733 et EP 1 069 142. Ces poudres sont généralement obtenues par polymérisation précipitante du (ou des) monomère(s) en solution dans un solvant organique type benzène, acétate d'éthyle, cyclohexane, tertio-butanol ; ce procédé nécessite donc de nombreuses étapes successives de purification du produit final, pour éliminer toute trace de solvant résiduel.

Parmi les latex inverses les plus connus on peut citer ceux commercialisés sous les noms Sepigel™, Simulgel™ et Sepiplus™. Ces épaississants sont obtenus par polymérisation radicalaire en émulsion inverse. Ils sont plus aisément manipulables, notamment à température ambiante, et se dispersent très rapidement dans l'eau. De plus, ils développent des performances épaississantes remarquablement élevées. Cependant, comme ils contiennent une huile, cela les rend potentiellement moins pertinents pour préparer des gels aqueux translucides ou transparents.

Il existe aussi des épaississants synthétiques ayant les performances épaississantes équivalentes ou supérieures aux latex inverses, mais présentant des possibilités d'usage plus polyvalentes, en particulier du fait de l'absence de toute phase huile pouvant conduire à des gels aqueux plus clairs. Ils se présentent sous la forme de poudres mais possèdent des temps de dissolution, et donc une facilité de mise en œuvre, comparable à ceux des produits se présentant sous la forme de liquides. Ils sont décrits dans la demande de brevet européen publiée sous le numéro EP 1 496 081 A2. Ils sont obtenus par les techniques classiques de polymérisation, telles que la polymérisation radicalaire en phase dispersée, la polymérisation radicalaire en suspension inverse, la polymérisation radicalaire en émulsion inverse ou en microémulsion inverse combinés suivies d'une séparation par différentes techniques telles que la précipitation dans un tiers solvant, la précipitation dans un tiers solvant puis éventuellement d'un lavage, d'un séchage par atomisation ou par déshydratation azéotropique, suivie éventuellement d'un lavage par un solvant judicieusement choisi. Ces épaississants synthétiques combinent donc une partie des avantages des épaississants synthétiques se présentant sous la forme de poudres classiques (absence d'huile, obtention de gels aqueux plus clairs) et les avantages des épaississants synthétiques se présentant sous la forme de latex inverse (pré-neutralisation, haute vitesse de dissolution, pouvoir épaississant et propriétés stabilisantes remarquables). Cependant pour des utilisations destinées à préparer des sérums tels que décrits précédemment, les clients utilisateurs de tels systèmes épaississants synthétiques souhaitent pouvoir fabriquer des gels encore plus clairs, que ceux obtenus aujourd'hui, voir des gels transparents. De plus, les gels obtenus avec ces épaississants synthétiques n'ont pas une stabilité satisfaisante lorsque la composition est riche en électrolytes comme c'est souvent le cas des sérums comprenant par exemple des extraits de végétaux riches en électrolytes, ou bien des sels minéraux.

Des terpolymères linéaires, branchés ou réticulés d'au moins un monomère possédant une fonction acide fort, libre, partiellement salifiée ou totalement salifiée, et au moins un monomère de formule (B) : 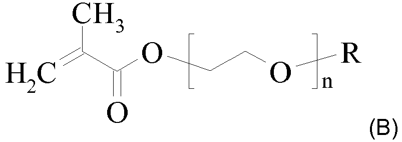 dans laquelle R représente un radical alkyle linéaire ou ramifié comportant de huit à vingt atomes de carbone et n représente un nombre supérieur ou égal à un et inférieur ou égal à trente sont décrits dans la demande internationale publiée sous le numéro WO 2011/030044,

Cependant, lorsque des gels aqueux sont préparés par la mise en œuvre de tels terpolymères épaississants synthétiques en présence d'une quantité supérieure à 0,2% massique d'électrolytes, on observe toujours un aspect qui ne satisfait pas à l'exigence de transparence liée à la préparation de sérums et souhaitée par les consommateurs.

Les inventeurs ont donc cherché à développer de nouveaux gels aqueux, stables au stockage, qui conservent une viscosité élevée en présence de milieux riches en électrolytes et sur une large gamme de pH, ainsi que des propriétés sensorielles satisfaisantes, à savoir exempte de caractère collant et filant lors de leur préhension et après application sur la peau, et qui présentent un aspect transparent.

Selon un premier aspect l'invention a pour objet, une composition (C₁) comprenant pour 100 % de sa masse :
a) - De 90% massique à 99% massique d'eau comprenant sous forme dissoute, entre 0,02 mole et 1 mole par litre d'au moins un sel (S) du cation ammonium ou d'un cation métallique monovalent ou multivalent (S) avec un anion organique ou inorganique,
b) - De 0,5% massique à 5% massique d'au moins un polyélectrolyte anionique réticulé (PA) issu de la polymérisation, en présence d'au moins un agent de réticulation, de l'acide 2-méthyl 2-[(1-oxo 2-propènyl) amino] 1-propanesulfonique partiellement ou totalement salifié, avec au moins un monomère neutre choisi parmi les N,N-dialkyl acrylamides, dans lesquels chacun des groupes alkyle comportent entre un et quatre atomes de carbones, et au moins un monomère de formule (I) : 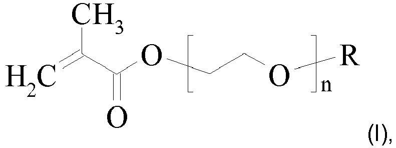 dans laquelle R représente un radical alkyle linéaire ou ramifié comportant de huit à vingt atomes de carbone et n représente un nombre supérieur ou égal à un et inférieur ou égal à vingt, et
c) - De 0,5% massique à 5% massique d'au moins un composé de formule (II) : 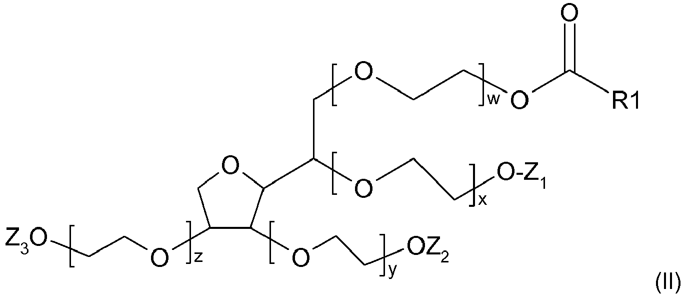 dans laquelle le radical R₁-C(=O) représente un radical acyle, saturé ou insaturé, linéaire ou ramifié, éventuellement substitué avec un ou plusieurs groupe hydroxyle, comportant de seize à vingt-deux atomes de carbone, Z₁, Z₂ et Z₃, identiques ou différents, représentent soit l'atome d'hydrogène, soit le radical R₁-(C=O), x, y, z et w représentent chacun un nombre supérieur ou égal à un et inférieur ou égal à quarante, tels que la somme (S_{M}) = x+y+z+w soit supérieure ou égale à quatre et inférieure ou égale à quarante.

étant entendu que dans ledit composition (C₁), le rapport massique (Rₐ) entre ledit polyélectrolyte anionique réticulé (PA) et ledit composé de formule (II) est supérieur ou égal à 0,2 et inférieur ou égal à 2,0 et le rapport molaire (R_{b}) entre le composé de formule (II) et le sel (S) est supérieur ou égal à 0,02 et inférieur ou égal à 1.

Par polyélectrolyte anionique réticulé (PA), on désigne, dans la définition de la composition (C₁) objet de la présente invention, un polyélectrolyte anionique réticulé non linéaire, se présentant à l'état de réseau tridimensionnel insoluble dans l'eau, mais gonflable à l'eau et conduisant à l'obtention d'un gel chimique.

Par partiellement salifié ou totalement salifié, on signifie dans la définition du polyélectrolyte anionique réticulé (PA) présent dans la composition (C₁) telle que définie ci-dessus, que ledit acide 2-méthyl 2-[(1-oxo 2-propènyl) amino] 1-propanesulfonique est partiellement ou totalement salifié, notamment sous forme de sel de métal alcalin, par exemple sous forme de sel de sodium ou de sel de potassium, ou sous forme de sel d'ammonium.

Par monomère neutre choisi parmi les N,N-dialkyl acrylamide, dans lesquels chacun des groupes alkyle comportent entre un et quatre atomes de carbones, on désigne, dans la définition de la composition (C₁) objet de la présente invention, plus particulièrement le N,N-diméthyl acrylamide, le N,N-diéthyl acrylamide, le N,N-dipropyl acrylamide ou le N,N-di-isopropyl acrylamide.

Selon un aspect particulier de la présente invention, dans la composition (C₁) telle que définie ci-dessus, ledit polyélectrolyte anionique réticulé (PA) comporte pour 100% molaire de ses monomères constitutifs, de 5% molaire à 95% molaire d'unités monomériques issues de l'acide 2-méthyl 2-[(1-oxo 2-propènyl) amino] 1-propanesulfonique partiellement ou totalement salifié, plus particulièrement de 10% molaire à 90% molaire, et tout particulièrement de 20% molaire à 80% molaire.

Selon un autre aspect particulier de la présente invention, dans la composition (C₁) telle que définie ci-dessus, ledit polyélectrolyte anionique réticulé (PA) comporte pour 100% molaire de ses monomères constitutifs, de 4,9% molaire à 90% molaire d'unités monomériques issues d'au moins un monomère neutre choisi parmi les N,N-dialkyl acrylamide, dans lesquels chacun des groupes alkyle comportent entre un et quatre atomes de carbones, plus particulièrement de 9,5% molaire à 85% molaire, et tout particulièrement de 15% molaire à 75% molaire.

Selon un autre aspect particulier de la présente invention, dans la composition (C₁) objet de la présente invention, ledit polyélectrolyte anionique réticulé (PA) comporte pour 100% molaire de ses monomères constitutifs, de 0,1% molaire à 10% molaire et plus particulièrement de 0,5% molaire à 5% molaire, d'unités monomériques issues du monomère de formule (I), telle que définie précédemment.

L'invention a plus particulièrement pour objet une composition (C₁) telle que définie précédemment, caractérisée en ce que ledit polyélectrolyte anionique réticulé (PA) comporte pour 100% molaire de ses monomères constitutifs :
- De 20% molaire à 80% molaire d'unités monomériques issues de l'acide 2-méthyl 2-[(1-oxo 2-propènyl) amino] 1-propanesulfonique partiellement ou totalement salifié
- De 15% molaire à 75% molaire d'unités monomériques issues d'un monomère neutre choisi parmi les N,N-dialkyl acrylamide, dans lesquels chacun des groupes alkyle comportent entre un et quatre atomes de carbones;
- De 0,5% à 5% molaire d'unités monomériques issues d'un monomère de formule (I) telle que définie précédemment.

Selon un aspect encore plus particulier, la composition (C₁) telle que définie précédemment, est caractérisée en ce que ledit monomère neutre est le N,N-diméthyl acrylamide.

Dans la formule (I) telle que définie précédemment, par radical alkyle linéaire ou ramifié comportant de huit à vingt atomes de carbone, on désigne plus particulièrement pour R :
- Ou bien un radical dérivé d'un alcool primaire linéaire, par exemple choisi parmi les radicaux octyle, décyle, undécyle, dodécyle, tridécyle, tétradécyle, pentadécyle, hexadécyle, heptadécyle, octadécyle, nonadécyle ou eicosyle ;
- Ou bien un radical dérivé d'un alcool de Guerbet, qui sont des 1-alcanols ramifiés répondant à la formule générale :

   CH₃-(CH₂)ₚ-CH[CH₃-(CH₂)ₚ₋₂]-CH₂OH,

   dans laquelle p représente un nombre entier compris supérieur à égal trois et inférieur ou égal à neuf, par exemple choisi parmi les radicaux 2-éthyl hexyle, 2-propyl heptyle, 2-butyl octyle, 2-pentyl nonyle, 2-hexyl décyle ou 2-octyl dodécyle ;
- Ou bien un radical dérivé d'un isoalcanol répondant à la formule générale :

   CH₃-CH(CH₃)-(CH₂)ₘ-CH₂OH,

   dans laquelle m représente un nombre entier supérieur ou égal à quatre et inférieur ou égal à seize, par exemple choisi parmi les radicaux 6-méthyl heptyle, 15-méthyl pendadécyle ou 16-méthyl heptadécyle,
- Ou bien un radical dérivé d'un alcool primaire ramifié, par exemple choisi parmi les radicaux 2-hexyl octyle, 2-octyl décyle ou 2-hexyl dodécyle.

Selon un autre aspect particulier, l'invention a pour objet une composition (C₁) telle que définie précédemment, caractérisée en ce que dans la définition de la formule (I), R représente un radical alkyle comportant de douze à dix-huit atomes de carbone et plus particulièrement un radical choisi parmi les radicaux dodécyle, tridécyle, tétradécyle, hexadécyle, heptadécyle et octadécyle.

Selon un autre aspect particulier, l'invention a pour objet une composition (C₁) telle que définie précédemment, caractérisée en ce que dans la définition de la formule (I), n représente un nombre entier supérieur ou égal à trois et inférieur ou égal à vingt.

Selon un aspect encore plus particulier, la composition (C₁) telle que définie précédemment, est caractérisée en ce que ledit monomère de formule (I) est le méthacrylate de lauryle tétraéthoxylé.

Selon un autre aspect encore plus particulier, la composition (C₁) telle que définie précédemment, est caractérisée en ce que ledit monomère de formule (I) est le méthacrylate de stéaryle eicosa-éthoxylé.

Selon un autre aspect particulier, l'invention a pour objet une composition (C₁) telle que définie précédemment, caractérisée en ce que l'agent de réticulation mis en œuvre dans la polymérisation de laquelle est issu ledit polyélectrolyte anionique réticulé (PA) tel que défini précédemment, est un composé diéthylénique ou polyéthylènique mis en œuvre dans une proportion molaire exprimée par rapport aux monomères mis en œuvre, supérieure ou égale à 0,005% et inférieure ou égale à 1% molaire, plus particulièrement supérieure ou égale à 0,01% et inférieure ou égale à 0,5% molaire et tout particulièrement supérieure ou égale à 0,01% et inférieure ou égale à 0,25% molaire. L'agent de réticulation est plus particulièrement choisi parmi le diméthacrylate d'éthylèneglycol, le tétraallyloxyéthane, le diacrylate d'éthylèneglycol, le diallyl urée, le triallyl amine, le propanetriacrylate de triméthylole ou le méthylène-bis(acrylamide) ou un mélange de ces composés et tout particulièrement choisi parmi le triallyl amine, le propanetriacrylate de triméthylol ou le méthylène-bis(acrylamide).

Dans la réaction de polymérisation conduisant au polyélectrolyte anionique réticulé (PA) mis en œuvre dans la composition (C₁) objet de la présente invention, on aussi mettre en œuvre divers additifs tels que des agents complexants, des agents de transfert ou des agents limiteurs de chaîne. Les agents de transfert ou limiteurs de chaîne sont plus particulièrement choisi parmi le groupe constitué par l'hypophosphite de sodium, des alcools de faibles poids moléculaires par exemple le méthanol, l'éthanol, le propanol-1, l'isopropanol, le butanol, des thiols, par exemple le 2-mercapto éthanol, des agents de transfert comprenant une fonction sulfate, par exemple le methallylsulfonate de sodium, ou des mélanges desdits agents de transfert. Les agents de transfert ou limiteurs de chaînes sont plus particulièrement utilisés dans des proportions molaires, exprimées par rapport au nombre total de moles de monomères mis en œuvre, supérieure ou égale à 0,001% et inférieure ou égale à 1% molaire, plus particulièrement supérieure ou égale à 0,001% et inférieure ou égale à 0,5% molaire, et tout particulièrement supérieure ou égale à 0,001 % et inférieure ou égale à 0,1% molaire.

Dans la formule (II) telle que définie précédemment, par radical acyle, saturé ou insaturé, linéaire ou ramifié, comportant de de seize à vingt-deux atomes de carbone, éventuellement substitué avec un ou plusieurs groupes hydroxyle, on désigne plus particulièrement pour R₁-C(=O) :
- Ou bien un radical acyle linéaire et saturé, par exemple choisi parmi les radicaux hexadécanoyle, heptadécanoyle, octadécanoyle, nonadécanoyle, eicosanoyle ou docosanoyle ;
- Ou bien un radical acyle linéaire insaturé, par exemple choisi parmi les radicaux hexadécènoyle, heptadécènoyle, octadécènoyle, cis-octadéc-9-énoyle (ou oléyle), (9Z,12Z)-octadéca-9,12-diènoyle (ou linoléyle), (9Z,12Z,15Z)-octadéca-9,12,15-triènoyle (ou α-linolénoyle), nonadécènoyle, eicosènoyle et docosènoyle ;
- Ou bien un radical acyle saturé ou insaturé, linéaire ou ramifié, comportant de seize à vingt-deux atomes de carbone substitué par un ou deux groupes hydroxy, par exemple choisi parmi les radicaux hydroxyhexadécanoyle, hydroxyoctadéanoyle, comme le radical 12-hydroxy octadécanoyle.

Selon un autre aspect particulier, l'invention a pour objet une composition (C₁) telle que définie précédemment, caractérisée en ce que dans la définition de la formule (II), le radical acyle R₁-C(=O) un radical est choisi parmi les radicaux hexadécanoyle, octadécanoyle, octadécènoyle, cis-octadéc-9-énoyle (ou oléyle), (9Z,12Z)-octadéca-9,12-diènoyle (ou linoléyle), (9Z,12Z,15Z)-octadéca-9,12,15-triènoyle (ou α-linolénoyle), eicosanoyle ou docosanoyle et est plus particulièrement choisi parmi les radicaux hexadécanoyle, octadécanoyle ou cis-octadéc-9-énoyle (ou oléyle).

Selon un autre aspect plus particulier, l'invention a pour objet une composition (C₁) telle que définie précédemment, caractérisée en ce que dans la formule (II), soit Z₁, Z₂ et Z₃, sont identiques et représentent l'atome d'hydrogène ; soit Z₁ et Z₂ sont identiques et représentent l'atome d'hydrogène et Z₃ représente le radical R₁-C(=O) ; soit Z₁ et Z₃ sont identiques et représentent l'atome d'hydrogène et Z₂ représente le radical R₁-C(=O), soit Z₂ et Z₃ sont identiques et représentent l'atome d'hydrogène et Z₁ représente le radical R₁-C(=O) ; soit Z₁ et Z₂ sont identiques et représentent le radical R₁-C(=O) et Z₃ représente l'atome d'hydrogène ; soit Z₁ et Z₃ sont identiques et représentent le radical R₁-C(=O) et Z₂ représente l'atome d'hydrogène ; soit Z₂ et Z₃ sont identiques, représentent le radical R₁-C(=O), et Z₁ représente l'atome d'hydrogène ; ou soit Z₁, Z₂ et Z₃ sont identiques et représentent le radical R₁-C(=O).

Selon un aspect plus particulier, l'invention a pour objet une composition (C₁) telle que définie précédemment, caractérisée en ce que la somme (S_{M}) = x+y+w+z est supérieure ou égale à 5 et inférieure ou égal à 40, plus particulièrement supérieure ou égale à 5 et inférieur ou égal à 20, plus particulièrement supérieure ou égale à 10 et inférieure ou égale à 20, et encore plus particulièrement égale à 20.

Selon un autre aspect particulier, l'invention a pour objet une composition (C₁) telle que définie précédemment, caractérisée en ce que dans la formule (II), ou bien le radical R₁-C(=O) représente un radical choisi parmi les radicaux hexadécanoyle, octadécanoyle, ou cis-octadéc-9-ènoyle (ou oléyle), Z₁, Z₂ et Z₃ sont identiques et représentent l'atome d'hydrogène, et la somme (S_{M}) = x+y+w+z est égale à 20 ; ou bien le radical R₁-C(=O) représente le radical cis-octadéc-9-énoyle (ou oléyle), Z₁, Z₂ et Z₃ sont identiques, représentent l'atome d'hydrogène, et la somme (S_{M}) = x+y+w+z est égale à 20 ; ou bien le radical R₁-C(=O) représente le radical octadécanoyle, Z₁, Z₂ et Z₃ sont identiques et représentent l'atome d'hydrogène, et la somme (S_{M}) = x+y+w+z est égale 20 ; ou bien le radical R₁-C(=O) représente le radical hexadécanoyle, Z₁, Z₂ et Z₃ sont identiques et représentent l'atome d'hydrogène, et la somme (S_{M}) = x+y+w+z est égale à 20 ; ou bien le radical R₁-C(=O) représente le radical cis-octadéc-9-ènoyle (ou oléyle), Z₂ et Z₃ sont identiques et représentent le radical cis-octadéc-9-énoyle (ou oléyle), Z₁ représente l'atome d'hydrogène, et la somme (S_{M}) = x+y+w+z est égale à 20.

Selon un aspect tout particulier, l'invention a pour objet une composition (C₁) telle que définie précédemment, caractérisée en ce que dans la formule (II), le radical R₁-C(=O) représente représente le radical cis-octadéc-9-énoyle (ou oléyle), Z₁, Z₂ et Z₃ sont identiques et représentent l'atome d'hydrogène, et la somme (S_{M}) = x+y+w+z est égale à 20.

Selon un aspect particulier, l'invention a pour objet une composition (C₁) telle que définie précédemment, caractérisée en ce que ledit polyélectrolyte anionique réticulé (PA) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

Comme exemple tout particulier d'une telle composition, il y a la composition (C₁) telle que définie précédemment, caractérisée en ce que ledit polyélectrolyte anionique réticulé (PA) comporte pour 100% molaire :
- De 60% molaire à 80% molaire d'unités monomériques issues de l'acide 2-méthyl 2-[(1-oxo 2-propènyl) amino] 1-propanesulfonique partiellement salifié sous forme d'ammonium,
- De 15% molaire à 39,5% molaire d'unités monomériques issues du N,N-diméthyl acrylamide, et
- De 0,5 % molaire à 5% molaire d'unités monomériques issues du méthacrylate de lauryle tétraéthoxylé.

Dans la composition (C₁) telle que définie précédemment et objet de la présente invention, par sel (S) on désigne un composé hétéropolaire dont le réseau cristallin comprend la participation d'au moins un type de cation différent des ions hydrogène et d'au moins un type d'anion différent des ions hydroxydes.

Selon un aspect particulier, le sel (S) se présentant sous une forme dissoute dans la composition (C₁) objet de la présente invention, est sélectionné parmi les sels inorganiques et parmi les sels organiques.

Selon un plus aspect particulier, l'invention a pour objet une composition (C₁) telle que définie précédemment caractérisée en ce que le sel (S) est choisi parmi les sels inorganiques et plus particulièrement parmi les halogénures, les carbonates, les bicarbonates, les phosphates, les nitrates, les borates et les sulfates d'ammonium ou d'un cation métallique.

Selon un aspect plus particulier, l'invention a pour objet une composition (C₁) telle que définie précédemment caractérisée en ce que le sel (S) est un sel inorganique dont le cation métallique est un cation monovalent ou multivalent choisi parmi les cations du sodium, du potassium, du lithium, du calcium, du magnésium, du zinc, du manganèse, du fer, du cuivre, du cobalt, de l'argent, de l'or, de l'aluminium, du barium, du bismuth, du sélénium, du zirconium, du strontium et de l'étain.

Selon un autre aspect particulier, le sel (S) est particulièrement choisi parmi les sels organiques et plus particulièrement un sel (S) du cation ammonium ou d'un cation métallique monovalent ou multivalent (S) avec un anion organique possédant au moins une fonction carboxylate, sulfonate ou sulphate.

Selon cet aspect particulier, l'invention a pour objet une composition (C₁) telle que définie précédemment caractérisée en ce que le sel (S) est un sel organique constitué par un cation métallique monovalent ou multivalent plus particulièrement choisi parmi les éléments du groupe constitué par les cations du sodium, du potassium, du lithium, du calcium, du magnésium, du zinc, du manganèse, du fer, du cuivre, du cobalt de l'argent, de l'or, de l'aluminium, du barium, du bismuth, du sélénium, du zirconium, du strontium et de l'étain. Selon cet aspect particulier, le sel (S) est un sel organique constitué par le cation choisi parmi les éléments groupe constitué par les cations du sodium, du calcium, du magnésium, du zinc et du manganèse, et encore plus particulièrement le sel (S) est un sel organique constitué par le cation du sodium.

Selon un autre aspect particulier, l'invention a pour objet une composition (C1) telle que définie précédemment caractérisée en ce que le sel (S) est un sel organique constitué par un cation qui est l'ion ammonium ou un cation métallique tel que décrit précédemment, et par un anion organique qui est un composé organique possédant au moins une fonction acide carboxylique sous forme carboxylate choisi parmi les éléments du groupe constitué par l'acide glycolique, l'acide citrique, l'acide tartrique, l'acide salicylique, l'acide lactique, l'acide mandélique, l'acide ascorbique, l'acide pyruvique, l'acide fumarique, l'acide rétinoïque, l'acide benzoïque, l'acide kojique, l'acide ellagique, l'acide malique, l'acide gluconique, l'acide sorbique, l'acide galacturonique, l'acide proprionique, l'acide déhydroacétique, l'acide heptanoïque, l'acide 4-amino benzoïque, l'acide cinnamique, l'acide benzalmalonique, l'acide aspartique, l'acide glutamique, la glycine, la capryloyl glycine, l'undécylènoyl glycine, l'acide undécylènoïque, la phénylalanine, l'undécylènoyle phénylalanine, l'acide hyaluronique.

Selon un aspect encore plus particulier, l'invention a pour objet une composition (C₁) telle que définie précédemment caractérisée en ce que le sel (S) est choisi parmi le chlorure de sodium, le chlorure de calcium, le chlorure de magnésium, le sulfate de calcium, le sulfate d'ammonium, le carbonate de calcium, le sulfate de zinc, le sulfate de magnésium, le borate de sodium, le glycolate de sodium, le citrate de sodium, le salicylate de sodium, le lactate de sodium, le gluconate de sodium, le gluconate de zinc, le gluconate de manganèse, le gluconate de cuivre et l'aspartate de magnésium.

Selon un autre aspect particulier, l'invention a pour objet une composition (C₁) telle que définie précédemment caractérisée en ce que le sel (S) est un sel organique constitué par un cation qui est l'ion ammonium ou un cation métallique tel que décrit précédemment, et par un anion organique qui est un composé organique possédant au moins une fonction acide sulfonique sous forme sulfonate choisi parmi les éléments du groupe constitué par l'acide 2-phényl benzimidazole-5-sulphonique, les acides sulphoniques dérivés des benzophénones, comme par exemple l'acide 4-hydroxy 2-methoxy 5-(oxo-phénylméthyl) benzènesulphonique (ledit acide étant enregistré sous l'appellation Benzophénone-4)" les acides sulphoniques dérivés du 3-benzylidene camphre comme par exemple l'acide 4-(2-oxo 3-bornylideneméthyl) benzènesulphonique, l'acide 2-méthyl 5-(2-oxo 3-bornylideneméthyl) benzènesulphonique.

Selon un aspect encore plus particulier, l'invention a pour objet une composition (C₁) telle que définie précédemment caractérisée en ce que le sel (S) est un sel organique sélectionné parmi les éléments du groupe constitué par le 2-phényl benzimidazole-5-sulphonate de sodium et le 4-hydroxy 2-méthoxy 5-(oxo-phénylméthyl) benzènesulphonate de sodium.

L'acide 2-phényl benzimidazole-5-sulphonique est commercialisé notamment sous le nom de marque EUSOLEX™232. Le 4-hydroxy 2-m2thoxy 5-(oxo-phénylméthyl) benzènesulphonate de sodium est enregistré sous l'appellation benzophénone-5.

La composition (C₁) selon l'invention est préparée par mélange de ses constituants, sous agitation mécanique, à une vitesse d'agitation comprise entre cinquante tours/minute et cinq cents tours/minutes, plus particulièrement entre cinquante tours/minutes et cent tours/minutes, à une température comprise entre 20°C et 80°C, plus particulièrement entre 20°C et 60°C et encore plus particulièrement entre 20°C et 45°C.

La composition (C₁) objet de la présente invention peut être conditionnée sous forme pressurisée dans un dispositif aérosol ou dans un dispositif de type « flacon pompe », dans un dispositif muni d'une paroi ajourée par exemple une grille, dans un dispositif muni d'un applicateur a billes (dit"roll-on). Lorsqu'elle est conditionnée dans des flacons, la composition (C₁) selon l'invention telle que définie précédemment peut être appliquée sous la forme de fines gouttelettes au moyen de dispositifs de pressurisation mécanique ou à gaz propulseur. Parmi les agents propulseurs que l'on peut associer à la composition (C₁) selon l'invention, il y a les composés hydrofluorés, comme le dichlorodifluorométhane, le trichlorofluorométhane, le difluoréthane, l'isobutane, le butane, le propane.

La composition (C₁) telle que définie précédemment peut en outre comporter des excipients et/ou des principes actifs habituellement mis en œuvre dans le domaine des formulations à usage topique, en particulier cosmétiques, dermocosmétiques, pharmaceutiques ou dermo-pharmaceutique.

C'est pourquoi, selon un autre mode particulier, l'invention a pour objet une composition (C₁) telle que définie précédemment, caractérisée en ce qu'elle comprend en outre, un ou plusieurs composés auxiliaires choisis parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents opacificants, les agents nacrants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Parmi les solvants volatils que l'on peut associer à la composition (C₁) selon l'invention, il y a les alcools hydrosolubles et volatils, comme l'éthanol, l'isopropanol ou le butanol, et plus particulièrement l'éthanol, les solvants organiques comme le glycérol, le diglycérol, les oligomères du glycérol, le propanediol-1,2, le propanediol-1,3, le butanediol-1,3, le butanediol-1,4, l'hexanediol-1,6, le 2-méthyl pentanediol-2,4, le pentanediol-1,2, le 2-méthyl propanediol-1,3, le 2-méthyl pentanediol-2,4l (ou hexylèneglycol), le dipropylèneglycol, le xylitol, l'érythritol ou le sorbitol. Selon un aspect particulier, la composition (C₁) selon l'invention comprend pour 100% de sa masse jusqu'à 20% massique d'un ou de plusieurs solvants volatils choisis parmi, l'éthanol, l'isopropanol, le butanol, le propanediol-1,3, le butanediol-1,3, le 2-méthyl pentanediol-2,4, le xylitol et le sorbitol.

Selon un autre aspect particulier, la composition (C₁) selon l'invention ne comprend pas de solvants volatils.

Selon un autre aspect particulier, la composition (C₁) selon l'invention est un hydrogel.

Parmi les agents antioxydants hydrosolubles que l'on peut associer à la composition (C₁) selon l'invention, il y a l'acide ascorbique, la glutathione, l'acide tartrique, l'acide oxalique, le tétra sodium glutamate diacétate.

Parmi les agents séquestrants hydrosolubles que l'on peut associer à la composition (C₁) selon l'invention, il y a les sels de l'acide éthylènediamine tétracétique (EDTA), comme le sel de sodium de l'EDTA, les sels de l'acide diéthylènetriamine pentacétique (DTPA) comme les sels de sodium du DTPA.

Parmi les colorants hydrosolubles que l'on peut associer à la composition (C₁) selon l'invention, il y a le caramel, le Yellow 5, l'Acid Blue 9/ Blue 1, le Green 5, le Green 3 / Fast Green FCF 3, l'Orange 4, le Red 4 / Food Red 1, le Yellow 6, l'Acid Red 33 / Food Red 12, le Red 40, le carmine de cochenille (Cl 15850, Cl 75470), l'Ext. Violet 2, le Red 6-7, le Ferric Ferrocyanide, les Ultramarines, l'Acide Yellow 3 / Yellow 10, l'Acid Blue 3, le Yellow 10.

Parmi les agents hydrosolubles stabilisant de la couleur que l'on peut associer à la composition (C₁) selon l'invention, il y a le citrate de Tris(tétraméthyl hydroxy pipéridinol), benzotriazolyl butylphénol sulfonate de sodium, le benzotriazolyl dodecyl p-crésol.

Comme exemples de tensioactifs moussants et/ou détergents, éventuellement présents dans la composition (C₁) objet de la présente invention, on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer à la composition (C₁) objet de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents éventuellement présents dans la composition (C₁) objet de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents éventuellement présents dans la composition (C₁) objet de la présente invention, on peut citer particulièrement les dérivés d'ammoniums quaternaires.

Parmi les tensioactifs non ioniques moussants et/ou détergents éventuellement présents dans la composition (C₁) objet de la présente invention, on peut citer plus particulièrement les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah, les N-alkylamines.

Comme exemples d'agents de texture éventuellement présents dans la composition (C₁) objet de la présente invention, on peut citer des dérivés N-acylés d'acides aminés, par exemple la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV 14, les fibres de cellulose, les fibres de coton, les fibres de chitosane, le talc, la séricite, le mica, la perlite.

Comme exemples de principes actifs éventuellement présents dans la composition (C₁) objet de la présente invention, il ya :
- Les vitamines et leurs dérivés, par exemple, le rétinol (vitamine A) et ses esters (palmitate de rétinyle par exemple), l'acide ascorbique (vitamine C) sous forme de sel et ses esters, les dérives de sucre de l'acide ascorbique (par exemple l'ascorbyl glucoside), le tocophérol (vitamine E) et ses esters (par exemple l'acétate de tocophérol), les vitamines B3 ou B10 (niacinamide et ses dérivés) ;
- Les composés ayant une action éclaircissante ou dépigmentante de la peau, par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™ ;
- Les composés ayant une action apaisante comme le SEPICALM™ S, l'allantoïne et le bisabolol ;
- Les agents anti-inflammatoires ;
- Les composés montrant une action hydratante par exemple le diglycérol, le triglycérol, l'urée, les hydroxyurées, le glycérolglucoside, le diglycérolglucoside, les polyglycérylglucosides, l'érythritylglucoside, le sorbitylglucoside, le xylitylglucoside, la composition commercialisée sous le nom de marque AQUAXYL™ comprenant du xylitylglucoside, de l'anhydroxylitol et du xyllitol ;
- Les composés montrant une action amincissante ou lipolytique comme la caféine ou ses dérivés, l'ADIPOSLIM™, l'ADIPOLESS™ ;
- Les protéines N-acylées ; les peptides N-acylés par exemple le MATRIXIL™ ; les acides aminés N-acylés ; les hydrolysâts partiels de protéines N-acylés ; les acides aminés ; les peptides ; les hydrolysâts totaux de protéines ;
- Les extraits végétaux riches en tanins en polyphénols et/ ou en isoflavones, par exemple les extraits de raisin, les extraits de pin, les extraits de vin, les extraits d'olives ; les extraits de soja, par exemple la Raffermine™ ; les extraits de blé par exemple la TENSINE™ ou la GLIADINE™ ; les extraits végétaux riches en terpènes ; les extraits d'algues d'eau douce ou marines ; les extraits marins en général comme les coraux ;
- Les composés ayant une action antimicrobienne ou une action purifiante, par exemple le LIPACIDE™ C8G, le LIPACIDE™ UG, le SEPICONTROL™ A5 ; l'OCTOPIROX™ ou le SENSIVA™ SC50 ;
- Les composés ayant une propriété énergisante ou stimulante comme le Physiogényl™, le panthénol et ses dérivés comme le SEPICAP™ MP ;
- Les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ;
- Les actifs anti-photo vieillissement ;
- Les actifs augmentant la synthèse des composants de la matrice extracellulaire par exemple le collagène, les élastines, les glycosaminoglycanes ;
- Les actifs agissant favorablement sur la communication cellulaire chimique comme les cytokines ou physiques comme les intégrines ;
- Les actifs créant une sensation de « chauffe » sur la peau comme les activateurs de la microcirculation cutanée (par exemple les dérivés de l'acide nicotinique) ou des produits créant une sensation de « fraîcheur » sur la peau (par exemple le menthol et des dérivés) ;
- Les actifs améliorant la microcirculation cutanée, par exemple les veinotoniques ; les actifs drainants ; les actifs à visée décongestionnante par exemple les extraits de ginko biloba, de lierre, de marron d'inde, de bambou, de ruscus, de petit houx, de *centalla asiatica,* de fucus, de romarin, de saule ;
- les actifs agissant comme agents tenseurs de la peau, par exemple les hydrolysats de protéines végétales, les hydrolysats d'origine marine comme les hydrolysats d'extraits de laminaires, les hydrolysats de cartilages de poissons, l'élastine marine, le produit commercialisé par la société SEPPIC sous le nom de marque SESAFLASH™, les solutions de collagène.
- Les agents de bronzage ou de brunissement de la peau, par exemple la dihydroxyacétone, l'isatine, l'alloxane, la ninhydrine, le glycéraldéhyde, l'aldéhyde mésotartrique, la glutaraldéhyde, l'érythrulose.

Comme exemples d'agents déodorants éventuellement présents dans la composition (C₁) objet de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le Triclosan™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples de filtres solaires éventuellement présents dans la composition (C₁) objet de la présente invention, on peut citer tous ceux figurant dans la directive cosmétique 76/768/CEE modifiée annexe VII.

L'invention a aussi pour objet l'utilisation de la composition (C₁) telle que précédemment, en tant de composition cosmétique topique, pour le nettoyage et/ou la protection et/ou le soin de la peau et/ou pour améliorer et/ou pour préserver l'aspect esthétique de la peau, des cheveux ou des muqueuses.

Le mot "topique" utilisé dans la définition de la composition (C₁), signifie au sens de la présente demande que ladite composition (C₁) est mise en œuvre par application sur la peau, le cuir chevelu ou les muqueuses, qu'il s'agisse d'une application directe dans le cas d'une composition cosmétique, dermocosmétique, dermo-pharmaceutique ou pharmaceutique ou d'une application indirecte par exemple dans le cas d'un produit d'hygiène corporelle, de soin ou de protection de la peau, se présentant sous la forme d'un article en textile, comme par exemple une lingette, ou en papier, comme par exemple un papier à usage sanitaire.

Selon un aspect plus particulier, lorsque la composition (C₁) objet de la présente invention est utilisée pour le nettoyage de la peau, des cheveux ou du cuir chevelu, elle comprend en outre au moins un tensioactif détergent.

Selon un autre aspect plus particulier, la composition (C₁) objet de la présente invention, peut être utilisée pour le soin ou pour la protection de la peau, par exemple du rayonnement solaire, ou pour prévenir le vieillissement cutané ou encore comme produit de traitement cosmétique de l'acné et/ou des points noirs et/ou des comédons ou comme produit hydratant.

L'invention a aussi pour objet un procédé pour éclaircir une composition comprenant au moins 90% massique d'eau, ladite eau comprenant sous forme dissoute entre 0,02 mole et 1 mole par litre d'au moins un sel (S) du cation ammonium ou d'un cation métallique monovalent ou multivalent (S) avec un anion organique ou inorganique et au moins un polyélectrolyte anionique réticulé (PA) issu de la polymérisation, en présence d'au moins un agent de réticulation, de l'acide 2-méthyl 2-[(1-oxo 2-propènyl) amino] 1-propanesulfonique partiellement ou totalement salifié, avec au moins un monomère neutre choisi parmi les N,N-dialkyl acrylamides, dans lesquels chacun des groupes alkyle comportent entre un et quatre atomes de carbones, et au moins un monomère de formule (I) : 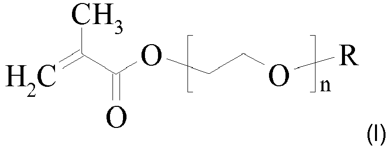 dans laquelle R représente un radical alkyle linéaire ou ramifié comportant de huit à vingt atomes de carbone et n représente un nombre supérieur ou égal à un et inférieur ou égal à vingt,
**ledit procédé comprenant** une étape d'addition au sein de ladite composition, d'une quantité efficace d'au moins un composé de formule (II) : 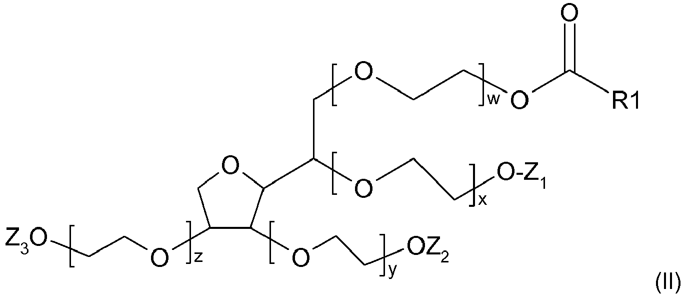 dans laquelle le radical R₁-C(=O) représente un radical acyle, saturé ou insaturé, linéaire ou ramifié, éventuellement substitué avec un ou plusieurs groupe hydroxyle, comportant de seize à vingt-deux atomes de carbone, Z₁, Z₂ et Z₃, identiques ou différents, représentent soit l'atome d'hydrogène, soit le radical R₁-(C=O), x, y, z et w représentent chacun un nombre supérieur ou égal à un et inférieur ou égal à quarante, tels que la somme (S_{M}) = x+y+z+w soit supérieure ou égale à quatre et inférieure ou égale à quarante.

Par « éclaircir », on entend au sens de la présente invention une modification visuelle de l'aspect de ladite composition, se traduisant par l'obtention d'une valeur de la turbidité de la composition (C₁) inférieure ou égale à 25 NTU, mesurée à une température de 25°C et à l'aide d'un turbidimètre optique de modèle DRT 100B commercialisé par la société HF Scientific, préalablement étalonné par une solution de Formazine (0,9 NTU).

Par « quantité efficace du composé de formule (II) », on désigne dans la définition du procédé tel que défini ci-dessus, une quantité massique du composé de formule (II) telle que la composition finale obtenue par ledit procédé :
- présente une valeur de turbidité inférieure ou égale à 25 NTU, mesurée à une température de 25°C et à l'aide d'un turbidimètre optique de modèle DRT 100B commercialisé par la société HF Scientific, préalablement étalonné par une solution de Formazine (0,9 NTU), et
- présente une viscosité dynamique supérieure ou égale à 100 mPa.s et inférieure ou égale à 50 000 mPa.s, ladite viscosité dynamique étant mesurée à l'aide d'un viscosimètre de marque Brookfield, de type LV, à une vitesse de rotation de 6 tours/minute et à une température de 25°C± 2°C, et
- reste homogène après un stockage de trois mois à une température de 25°C.

L'étude expérimentale suivante illustre l'invention sans toutefois la limiter.

### I) - Préparation de polvélectrolytes anioniques réticulés.

### I-1 Terpolymère de 2-méthyl 2-[(1-oxo 2-propènyl) amino] 1-propanesulfonate d'ammonium, de N,N-diméthyl acrylamide et de méthacrylate de lauryle tétraéthoxylé [AMPSNH₄/DMAM/MAL(4OE) 77,4/19,2/3,4 molaire], réticulé au propanetriacrylate de triméthylol (TMPTA).

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium (AMPSNH₄) dans un mélange tert-butanol/eau (97,5/2,5 en volume), 10,1g de N,N-diméthyl acrylamide (DMAM), 4,2g de méthacrylate de lauryle tétra-éthoxylé [MAL(4OE)] et 0,75g de TMPTA. Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, dénommé par la suite : "Polyélectrolyte (PA)".

### II) - Préparation et évaluation de compositions aqueuses selon l'invention et compositions aqueuses comparatives.

### II-1 Préparation d'une composition aqueuse selon l'invention

On prépare quatre compositions aqueuses selon l'invention, dénommées (E₁) à (E₄), dont les proportions massiques de leurs constituants sont indiquées dans le tableau 1.

Le procédé de préparation, commun à ces quatre compositions, est le suivant :
- on verse dans un réacteur muni d'une agitation mécanique et d'une double enveloppe comprenant un fluide caloporteur, à une température de 20°C, la quantité nécessaire d'eau qui est ensuite amenée à une température de 75°C sous agitation mécanique à une vitesse de quatre-vingt tours par minute ;
- le polyélectrolyte anionique réticulé (PA) est ensuite ajouté progressivement à cette température de 75°C sous agitation mécanique ;
- après homogénéisation du gel obtenu précédemment, l'agent solubilisant de formule (II) à tester est ensuite ajouté progressivement à la température de 75°C sous agitation mécanique à la vitesse de quatre-vingt tours par minute ;
- le mélange obtenu est ensuite refroidi à une température de 40°C, puis le sel (S) et l'agent conservateur sont ajoutés dans le milieu précédemment obtenu, toujours sous agitation mécanique à une vitesse de quatre-vingt tours par minute.

**Tableau 1**

| **Composition** | **(E₁)** | **(E₂)** | **(E₃)** | **(E₄)** |
|---|---|---|---|---|
| Polyélectrolyte (PA) (% massique) | 2,25 | 2,25 | 2,25 | 2,25 |
| Chlorure de sodium (S) (mole/litre) | 0,34 | 0,34 | 0,34 | 0,34 |
| Euxyl™ PE9010⁽¹⁾ (% massique) | 1 | 1 | 1 | 1 |
| MONTANOX™ 80²⁾ (II) (% massique) | 2,50 | 1,50 | 2,00 | 3,00 |
| Eau | Qs.100% | Qs.100% | Qs.100% | Qs.100% |
| Rₐ = (PA)/(II) (massique) | 0,90 | 1,5 | 1,13 | 0,75 |
| R_{b} = (II)/(S) (molaire) | 0.06 | 0.03 | 0.05 | 0.07 |

| | | | | |
|---|---|---|---|---|
| (1) Euxyl™ PE9010 est un mélange de Phenoxyethanol et Ethylhexylglycerine commercialisé par la société Schülke & Mayr ; (2) : MONTANOX™ 80 est un monooléate de sorbitan éthoxylé avec 20 moles d'oxyde d'éthylène commercialisé par la société SEPPIC (PM = 1308); | | | | |

### II-2 Préparation des compositions aqueuses comparatives

On prépare huit compositions aqueuses comparatives, dénommées (F₁) à (F₈), dont les proportions massiques de leurs constituants sont indiquées dans le tableau 2.

Le procédé de préparation, commun à ces huit compositions, est le suivant :
- on verse dans un réacteur muni d'une agitation mécanique et d'une double enveloppe comprenant un fluide caloporteur, à une température de 20°C, la quantité nécessaire d'eau qui est ensuite amenée à une température de 75°C sous agitation mécanique à une vitesse de 80 tours/minute ;
- le Polyélectrolyte anionique réticulé (PA) est ensuite ajouté progressivement à cette température de 75°C sous agitation mécanique ;
- après homogénéisation du gel obtenu précédemment, l'agent solubilisant de formule (II') à tester est ensuite ajouté progressivement à la température de 75°C sous agitation mécanique à la vitesse de quatre-vingt tours par minute ;
- le mélange obtenu est ensuite refroidi à une température de 40°C, puis le sel (S) et l'agent conservateur sont ajoutés dans le milieu précédemment obtenu, toujours sous agitation mécanique à une vitesse de quatre-vingt tours par minute.

**Tableau 2**

| **Composition** | **(F₁)** | **(F₂)** | **(F₃)** | **(F₄)** |
|---|---|---|---|---|
| Polyélectrolyte (PA) (% massique) | 2,25 | 2,25 | 2,25 | 2,25 |
| Chlorure de sodium (S) (mole/litre) | 0,34 | 0,34 | 0,34 | 0,34 |
| Euxyl™ PE9010⁽¹⁾ (% massique) | 1 | 1 | 1 | 1 |
| MONTANOX™ 20⁽³⁾ (II') (% massique) | 0 | 2,50 | 0 | 0 |
| SIMULSOL™ 1292⁽⁴⁾(II') (% massique) | 0 | 0 | 2,50 | 0 |
| ORAMIX™ CG 110⁽⁵⁾(II') (% massique) | 0 | 0 | 0 | 2,50 |
| Eau | Qs.100% | Qs.100% | Qs.100% | Qs.100% |
| R'ₐ = (PA)/(II') (massique) | Na | 0,9 | 0,9 | 0,9 |
| R'_{b} = (II')/(S) (molaire) | Nd | 0.06 | nd | Nd |

| | | | | |
|---|---|---|---|---|
| (3) : MONTANOX™ 20 est un monolaurate de sorbitan éthoxylé à 20 moles d'oxyde d'éthylène commercialisé par la société SEPPIC (PM = 1226) ; (4) : SIMULSOL™ 1292 (nom INCI : PEG-25 hydrogenated castor oil) est une huile de ricin hydrogénée et éthoxylée à 25 moles d'oxyde d'éthylène commercialisée par la société SEPPIC; (5) : ORAMIX™ CG 110 est une composition aqueuse comprenant du caprylyl glucoside et du capryl glucoside commercialisée par la société SEPPIC ; | | | | |

**Tableau 2 (Fin)**

| **Composition** | **(F₅)** | **(F₆)** | **(F₇)** | **(F₈)** |
|---|---|---|---|---|
| Polyélectrolyte (PA) (% massique) | 2,25 | 2,25 | 2,25 | 2,25 |
| Chlorure de sodium (S) (mole/litre) | 0,34 | 0,34 | 0,34 | 0,34 |
| Euxyl™ PE9010⁽¹⁾ (% massique) | 1 | 1 | 1 | 1 |
| SEPICLEAR™ G7⁽⁶⁾ (II')°(% massique) | 2,5 | 0 | 0 | 0 |
| MONTANOX™ 80⁽²⁾ (II) (% massique) | 0 | 0,5 | 1,0 | 5 |
| Eau | Qs.100% | Qs.100% | Qs.100% | Qs.100% |
| R'ₐ = (PA)/(II') (massique) | 0,9 | - | - | - |
| R'ₐ = (PA)/(II) (massique) | - | 4,5 | 2,25 | 0,45 |
| R'_{b} = (II')/(S) (molaire) | Nd | - | - | - |
| R_{b} = (II)/(S) (molaire) | - | 0.011 | 0.022 | 0.112 |

| | | | | |
|---|---|---|---|---|
| (6) : SEPICLEAR™ G7 est une composition aqueuse comprenant du n-heptyl glucoside | | | | |

### II-3 Mise en évidence des propriétés et des caractéristiques des compositions aqueuses selon l'invention et des compositions aqueuses comparatives

Les compositions aqueuses (E₁) à (E₄) selon l'invention et (F₁) à (F₈) comparatives ainsi préparées, sont ensuite conservées dans une enceinte climatique isolée et régulée à une température de 25°C pendant 7 jours. A l'issue de cette période de 7 jours, chaque composition aqueuse est évaluée par les mesures suivantes :
- Mesure de la viscosité dynamique (µ) à 25°C au moyen d'un viscosimètre de type Brookfield LVT à une vitesse de 6 tours/minutes (V6), muni du mobile adapté, après une période de stockage de 7 jours à 25°C.
- Mesure de la turbidité de la composition aqueuse obtenue à une température de 25°C, à l'aide d'un turbidimètre optique de marque HF Scientific™ et de modèle DRT100B, préalablement étalonné avec une solution de Formazine (0,9 NTU) ; les mesures de turbidité sont exprimées en unité NTU.
- Evaluation visuelle de l'aspect de la composition aqueuse préparée après une période de stockage de trois mois à 25°C ; l'aspect visuel de chaque composition aqueuse est noté par l'expérimentateur et qualifié de limpide (L), de trouble (T), d'opaque (O) ou de trouble-hétérogène (T-H), selon les cas.

Les résultats obtenus pour les compositions aqueuses (E₁) à (E₄) selon l'invention et (F₁) à (F₈) comparatives, sont consignés respectivement dans les tableaux 3 et 4 ci-après.

**Tableau 3**

| **Compositions** | **(E₁)** | **(E₂)** | **(E₃)** | **(E₄)** |
|---|---|---|---|---|
| Aspect visuel après 7 jours à 25°C | L | L | L | L |
| Aspect visuel après 3 mois à 25°C | L | L | L | L |
| µ après 7 jours à 25°C | 9.880 | 27.000 | 20.000 | 13.700 |
| Turbidité (NTU) | 20 | 9 | 23 | 16 |

**Tableau 4**

| **Compositions** | **(F₁)** | **(F₂)** | **(F₃)** |
|---|---|---|---|
| Aspect visuel après 7 jours à 25°C | T | T | T |
| Aspect visuel après 3 mois à 25°C | T | T | T |
| µ après 7 jours à 25°C | 58.000 | 15.000 | 9.500 |
| Turbidité (NTU) | >500 | >150 | >150 |

**Tableau 4 (fin)**

| **Compositions** | **(F₄)** | **(F₅)** | **(F₆)** | **(F₇)** | **(F₈)** |
|---|---|---|---|---|---|
| Aspect visuel après 7 jours à 25°C | T | T | T | O | T |
| Aspect visuel après 3 mois à 25°C | T | T | T | O | T |
| µ après 7 jours à 25°C | 20.000 | 25.500 | 41.900 | 27.000 | 10.700 |
| Turbidité (NTU) | >150 | 150 | 100 | 30 | 34 |

### II-4 Analyse des résultats

a) La mise en œuvre d'agents solubilisants connus de l'homme du métier, comme par exemple le Polysorbate 20, l'huile de ricin hydrogénée et éthoxylée à 25 moles d'oxyde d'éthylène, le caprylyl/capryl glucoside ou le n-heptyl glucoside, respectivement présent dans les compositions comparatives (F₂), (F₃), (F₄) et (F₅), ne permet pas d'éclaircir de façon suffisante et satisfaisante une composition aqueuse épaissie, comprenant une quantité massique de 2% de chlorure de sodium, représentée par la composition (F₁). En effet, les compositions comparatives (F₂), (F₃), (F₄) et (F₅), ne se distinguant que par la nature de l'agent solubilisant, ne permettent pas d'atteindre une valeur de la turbidité inférieure à 100 NTU.
b) L'utilisation comme agent solubilisant du monooméate de sorbitan éthoxylé avec 20 moles d'oxyde d'éthylène dans la compositions (E₁), permet d'éclaircir de façon suffisante et satisfaisante une composition aqueuse épaissie, comprenant une quantité massique de 2% de chlorure de sodium, représentée par la composition (F₁), alors que l'utilisation du monolaurate de sorbitan éthoxylé avec 20 moles d'oxyde d'éthylène dans la composition (F₆) ne permet pas d'obtenir un éclaircissement suffisant et satisfaisant. En effet, la composition selon l'invention (E₁) se caractérise par une valeur de turbidité respectivement de 20 NTU, alors que la turbidité de la composition comparative (F₂) est supérieure à 150 NTU.
c) Les compositions selon l'invention (E₂) à (E₄) montrent l'obtention de valeurs de turbidité inférieures à 25 NTU, pour différentes valeurs des rapports massique Rₐ et molaire R_{b} tels que définis précédemment, avec différentes proportions massiques des ingrédients concernés. Les compositions comparatives (F₆), (F₇) et (F₈) montrent que lorsque tous les paramètres requis ne sont pas réunis, l'éclaircissement procuré n'est pas suffisant et satisfaisant. En effet, dans le cas de la composition (F₆), la valeur du ratio massique Rₐ est établie à 4,5 et la valeur du ratio molaire R_{b} est établie à 0,25, et la valeur de la turbidité mesurée est de 43 NTU. Dans le cas de la composition (F₇), la valeur du ratio massique Rₐ est établie à 2,25 et la valeur du ratio massique R_{b} est établie à 0,5, et la valeur de la turbidité mesurée est de 28 NTU. Dans le cas de la composition (F₈), la valeur du ratio massique Rₐ est établie à 0,45 et la valeur du ratio massique R_{b}, est établie à 2,50 et la valeur de la turbidité mesurée est de 34 NTU.

La mise en perspective des résultats obtenus pour les compositions (E₁) à (E₄) selon l'invention et (F₁) à (F₆) comparatives, fait apparaître clairement que l'éclaircissement de compositions aqueuses épaissies et riches en sels, conservant un niveau suffisant de viscosité, ne pouvait pas se déduire des résultats associés aux compositions aqueuses de l'état de la technique.

## Revendications

1. Composition (C₁) comprenant pour 100 % de sa masse :
a) - De 90% massique à 99% massique d'eau comprenant sous forme dissoute, entre 0,02 mole et 1 mole par litre d'au moins un sel (S) du cation ammonium ou d'un cation métallique monovalent ou multivalent (S) avec un anion organique ou inorganique,
b) - De 0,5% massique à 5% massique d'au moins un polyélectrolyte anionique réticulé (PA) issu de la polymérisation, en présence d'au moins un agent de réticulation, de l'acide 2-méthyl 2-[(1-oxo 2-propènyl) amino] 1-propanesulfonique partiellement ou totalement salifié, avec au moins un monomère neutre choisi parmi les N,N-dialkyl acrylamides, dans lesquels chacun des groupes alkyle comportent entre un et quatre atomes de carbones, et au moins un monomère de formule (I) : 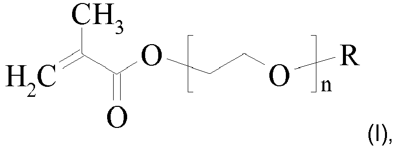 dans laquelle R représente un radical alkyle linéaire ou ramifié comportant de huit à vingt atomes de carbone et n représente un nombre supérieur ou égal à un et inférieur ou égal à vingt, et
c) - De 0,5% massique à 5% massique d'au moins un composé de formule (II) : 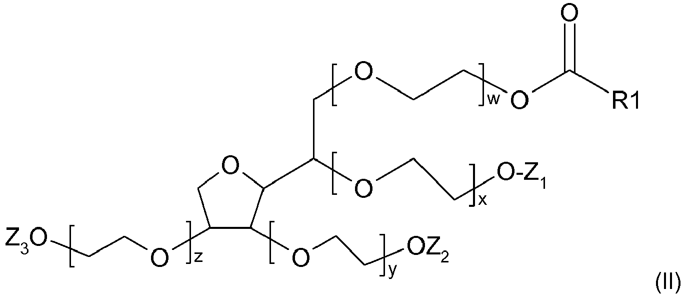 dans laquelle le radical R₁-C(=O) représente un radical acyle, saturé ou insaturé, linéaire ou ramifié, éventuellement substitué avec un ou plusieurs groupe hydroxyle, comportant de seize à vingt-deux atomes de carbone, Z₁, Z₂ et Z₃, identiques ou différents, représentent soit l'atome d'hydrogène, soit le radical R₁-(C=O), x, y, z et w représentent chacun un nombre supérieur ou égal à un et inférieur ou égal à quarante, tels que la somme (S_{M}) = x+y+z+w soit supérieure ou égale à quatre et inférieure ou égale à quarante.
étant entendu que dans ledit composition (C₁), le rapport massique (Rₐ) entre ledit polyélectrolyte anionique réticulé (PA) et ledit composé de formule (II) est supérieur ou égal à 0,2 et inférieur ou égal à 2,0 et le rapport molaire (R_{b}) entre le composé de formule (II) et le sel (S) est supérieur ou égal à 0,02 et inférieur ou égal à 1.

2. Composition (C₁) telle que définie à la revendication 1, **caractérisée en ce que** ledit polyélectrolyte anionique réticulé (PA) comporte, pour 100% molaire de ses monomères constitutifs :
- De 20% molaire à 80% molaire d'unités monomériques issues de l'acide 2-méthyl 2-[(1-oxo 2-propènyl) amino] 1-propanesulfonique partiellement ou totalement salifié
- De 15% molaire à 75% molaire d'unités monomériques issues d'un monomère neutre choisi parmi les N,N-dialkyl acrylamide, dans lesquels chacun des groupes alkyle comportent entre un et quatre atomes de carbones;
- De 0,5% à 5% molaire d'unités monomériques issues d'un monomère de formule (I) telle que définie précédemment.

3. Composition (C₁) telle définie à la revendication 2, **caractérisée en ce que** ledit monomère neutre est le N,N-diméthyl acrylamide.

4. Composition (C₁) telle que définie à l'une ou quelconque des revendications 1 à 3, **caractérisée en ce que** ledit monomère de formule (I) est le méthacrylate de lauryle tétraéthoxylé.

5. Composition (C₁) telle que définie à l'une ou quelconque des revendications 1 à 4, **caractérisée en ce que** dans la formule (II), le radical R₁-C(=O) représente représente le radical cis-octadéc-9-énoyle (ou oléyle), Z₁, Z₂ et Z₃ sont identiques et représentent l'atome d'hydrogène, et la somme (S_{M}) = x+y+w+z est égale à 20.

6. Composition (C₁) telle que définie à l'une ou quelconque des revendications 1 à 5, **caractérisée en ce que** ledit polyélectrolyte anionique réticulé (PA) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

7. Composition (C₁) telle que définie à l'une quelconque des revendications 1 à 6, **caractérisée en ce que** ledit polyélectrolyte anionique réticulé (PA) comporte pour 100% molaire :
- De 60% molaire à 80% molaire d'unités monomériques issues de l'acide 2-méthyl 2-[(1-oxo 2-propènyl) amino] 1-propanesulfonique partiellement salifié sous forme d'ammonium,
- De 15% molaire à 39,5% molaire d'unités monomériques issues du N,N-diméthyl acrylamide, et
- De 0,5 % molaire à 5% molaire d'unités monomériques issues du méthacrylate de lauryle tétraéthoxylé.

8. Composition (C₁) telle que définie à l'une ou quelconque des revendications 1 à 7, **caractérisé en ce que** ledit sel (S) est choisi parmi les halogénures, les carbonates, les bicarbonates, les phosphates, les nitrates, les borates et les sulfates d'ammonium ou d'un cation métallique.

9. Composition (C₁) telle que définie à l'une quelconque des revendications 1 à 8, **caractérisée en ce que** ledit sel (S) est un sel (S) du cation ammonium ou d'un cation métallique monovalent ou multivalent (S) avec un anion organique possédant au moins une fonction carboxylate, sulfonate ou sulphate.

10. Composition (C₁) telle que définie à l'une quelconque des revendications 1 à 9, **caractérisée en ce que** ledit sel (S) est choisi parmi le chlorure de sodium, le chlorure de calcium, le chlorure de magnésium, le sulfate de calcium, le sulfate d'ammonium, le carbonate de calcium, le sulfate de zinc, le sulfate de magnésium, le borate de sodium, le glycolate de sodium, le citrate de sodium, le salicylate de sodium, le lactate de sodium, le gluconate de sodium, le gluconate de zinc, le gluconate de manganèse, le gluconate de cuivre et l'aspartate de magnésium.

11. Composition (C₁) telle que définie à l'une quelconque des revendications 1 à 10, **caractérisée en ce qu'**elle comprend en outre, un ou plusieurs composés auxiliaires choisis parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents opacificants, les agents nacrants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

12. Utilisation de la composition (C₁) telle que définie à l'une ou quelconque des revendications 1 à 11, en tant de composition cosmétique topique, pour le nettoyage et/ou la protection et/ou le soin de la peau et/ou pour améliorer et/ou pour préserver l'aspect esthétique de la peau, des cheveux ou des muqueuses.

13. Procédé pour éclaircir une composition comprenant au moins 90% massique d'eau, ladite eau comprenant sous forme dissoute entre 0,02 mole et 1 mole par litre d'au moins un sel (S) du cation ammonium ou d'un cation métallique monovalent ou multivalent (S) avec un anion organique ou inorganique et au moins un polyélectrolyte anionique réticulé (PA) issu de la polymérisation, en présence d'au moins un agent de réticulation, de l'acide 2-méthyl 2-[(1-oxo 2-propènyl) amino] 1-propanesulfonique partiellement ou totalement salifié, avec au moins un monomère neutre choisi parmi les N,N-dialkyl acrylamides, dans lesquels chacun des groupes alkyle comportent entre un et quatre atomes de carbones, et au moins un monomère de formule (I) : 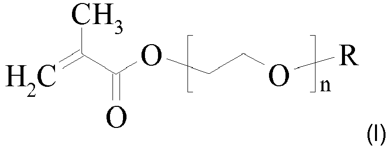 dans laquelle R représente un radical alkyle linéaire ou ramifié comportant de huit à vingt atomes de carbone et n représente un nombre supérieur ou égal à un et inférieur ou égal à vingt,
**ledit procédé comprenant** une étape d'addition au sein de ladite composition, d'une quantité efficace d'au moins un composé de formule (II) : 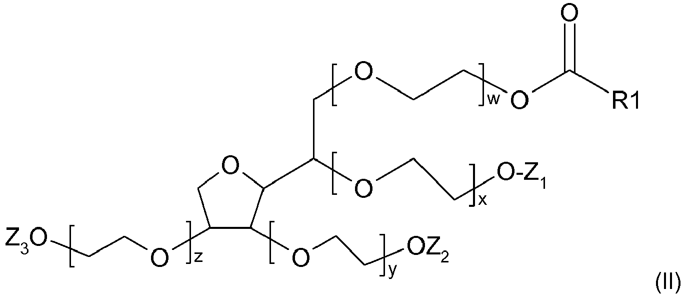 dans laquelle le radical R₁-C(=O) représente un radical acyle, saturé ou insaturé, linéaire ou ramifié, éventuellement substitué avec un ou plusieurs groupe hydroxyle, comportant de seize à vingt-deux atomes de carbone, Z₁, Z₂ et Z₃, identiques ou différents, représentent soit l'atome d'hydrogène, soit le radical R₁-(C=O), x, y, z et w représentent chacun un nombre supérieur ou égal à un et inférieur ou égal à quarante, tels que la somme (S_{M}) = x+y+z+w soit supérieure ou égale à quatre et inférieure ou égale à quarante.

## Patentansprüche

1. Zusammensetzung (C₁), für 100 % ihrer Masse Folgendes umfassend:
a) zu 90 Massen-% bis 99 Massen-% Wasser, umfassend in aufgelöster Form zwischen 0,02 Mol und 1 Mol je Liter mindestens ein Salz (S) des Ammoniumkations oder eines einwertigen oder mehrwertigen Metallkations (S) mit einem organischen oder anorganischen Anion,
b) zu 0,5 Massen-% bis 5 Massen-% von mindestens einem aus der Polymerisation stammenden vernetzten anionischen Polyelektrolyten (PA), in Gegenwart von mindestens einem Vernetzungsmittel, 2-Methyl-2-[(1-oxo-2-propenyl)amino]-1-propansulfonsäure, teilweise oder vollständig in Salzform vorliegend, mit mindestens einem neutralen Monomer, ausgewählt aus N,N-Dialkylacrylamiden, in denen jede der Alkylgruppen zwischen einem und vier Kohlenstoffatome und mindestens ein Monomer der Formel (I) beinhaltet: 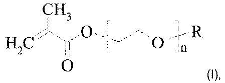 worin R einen linearen oder verzweigten Alkylrest repräsentiert, der von acht bis zwanzig Kohlenstoffatome beinhaltet und worin n eine Zahl größer als oder gleich eins und kleiner als oder gleich zwanzig repräsentiert, und
c) zu 0,5 Massen-% bis 5 Massen-% von mindestens einer Verbindung der Formel (II): 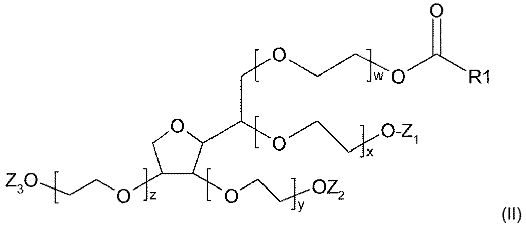 worin der Rest R₁-C(=O) einen gesättigten oder ungesättigten, linearen oder verzweigten, gegebenenfalls mit einer oder mehreren Hydroxylgruppen, die von sechzehn bis zweiundzwanzig Kohlenstoffatome beinhalten, substituierten Acylrest repräsentiert, wobei Z₁, Z₂ und Z₃, identisch oder unterschiedlich, entweder das Wasserstoffatom oder den Rest R₁-C(=O) repräsentieren, x, y, z und w jeweils eine Zahl größer als oder gleich eins und kleiner als oder gleich vierzig repräsentieren, sodass die Summe (S_{M}) = x+y+z+w größer als oder gleich vier und kleiner als oder gleich vierzig ist,
vorausgesetzt, dass in der Zusammensetzung (C₁) das Masseverhältnis (Rₐ) zwischen dem vernetzten anionischen Polyelektrolyten (PA) und der Verbindung der Formel (II) größer als oder gleich 0,2 und kleiner als oder gleich 2,0 ist, und das Molverhältnis (R_{b}) zwischen der Verbindung der Formel (II) und dem Salz (S) größer als oder gleich 0,02 und kleiner als oder gleich 1 ist.

2. Zusammensetzung (C₁) nach Anspruch 1, **dadurch gekennzeichnet, dass** der vernetzte anionische Polyelektrolyt (PA) für 100 Mol-% seiner konstitutiven Monomere beinhaltet:
- zu 20 Mol-% bis 80 Mol-% Monomereinheiten, die aus teilweise oder vollständig in Salzform vorliegenden der 2-Methyl-2-[(1-oxo-2-propenyl)amino]-1-propansulfonsäure stammen,
- zu 15 Mol-% bis 75 Mol-% Monomereinheiten, die aus einem neutralen Monomer stammen, das aus N,N-Dialkylacrylamid ausgewählt ist, worin jede der Alkylgruppen zwischen einem und vier Kohlenstoffatome beinhaltet;
- zu 0,5 Mol-% bis 5 Mol-% Monomereinheiten, die, wie zuvor definiert, aus einem Monomer der Formel (I) stammen.

3. Zusammensetzung (C₁) nach Anspruch 2, **dadurch gekennzeichnet, dass** das neutrale Monomer N,N-Dimethylacrylamid ist.

4. Zusammensetzung (C₁) nach einem der Ansprüche 1 bis 3, **dadurch gekennzeichnet, dass** das Monomer der Formel (I) tetraethoxyliertes Laurylmethacrylat ist.

5. Zusammensetzung (C₁) nach einem der Ansprüche 1 bis 4, **dadurch gekennzeichnet, dass** in der Formel (II) der Rest R₁-C(=O) den cis-Octadec-9-enoyl (oder -oleoyl)-Rest repräsentiert, Z₁, Z₂ und Z₃ identisch sind und das Wasserstoffatom repräsentieren, und die Summe (S_{M}) = x+y+w+z gleich 20 ist.

6. Zusammensetzung (C₁) nach einem der Ansprüche 1 bis 5, **dadurch gekennzeichnet, dass** der vernetzte anionische Polyelektrolyt (PA) ein Terpolymer von 2-Methyl-2-[(1-oxo-2-propenyl)amino]-1-propansulfonsäure, teilweise in Salzform in Form von Ammoniumsalz vorliegend, von N,N-Dimethylacrylamid und von tetraethoxyliertem Laurylmethacrylat ist, das mit Trimethylolpropantriacrylat vernetzt ist.

7. Zusammensetzung (C₁) nach einem der Ansprüche 1 bis 6, **dadurch gekennzeichnet, dass** der vernetzte anionische Polyelektrolyt (PA) für 100 Mol-% beinhaltet:
- zu 60 Mol-% bis 80 Mol-% Monomereinheiten, die aus teilweise in Salzform in Form von Ammonium vorliegender 2-Methyl-2-[(1-oxo-2-propenyl)amino]-1-propansulfonsäure stammen,
- zu 15 Mol-% bis 39,5 Mol-% Monomereinheiten, die aus dem N,N-Dimethylacrylamid stammen, und
- zu 0,5 Mol-% bis 5 Mol-% Monomereinheiten, die aus tetraethoxyliertem Laurylmethacrylat stammen.

8. Zusammensetzung (C₁) nach einem der Ansprüche 1 bis 7, **dadurch gekennzeichnet, dass** das Salz (S) aus den Halogeniden, den Carbonaten, den Bi-Carbonaten, den Phosphaten, den Nitraten, den Boraten und den Sulfaten von Ammonium oder einem Metallkation ausgewählt wird.

9. Zusammensetzung (C₁) nach einem der Ansprüche 1 bis 8, **dadurch gekennzeichnet, dass** das Salz (S) ein Salz (S) des Ammoniumkations oder eines einwertigen oder mehrwertigen Metallkations (S) mit einem organischen Anion ist, das mindestens eine Carboxylat-, Sulfonat- oder Sulfatfunktion aufweist.

10. Zusammensetzung (C₁) nach einem der Ansprüche 1 bis 9, **dadurch gekennzeichnet, dass** das Salz (S) aus Natriumchlorid, Kalziumchlorid, Magnesiumchlorid, Kalziumsulfat, Ammoniumsulfat, Kalziumcarbonat, Zinksulfat, Magnesiumsulfat, Natriumborat, Natriumglycolat, Natriumcitrat, Natriumsalicylat, Natriumlactat, Natriumgluconat, Zinkgluconat, Mangangluconat, Kupfergluconat und Magnesiumaspartat ausgewählt ist.

11. Zusammensetzung (C₁) nach einem der Ansprüche 1 bis 10, **dadurch gekennzeichnet, dass** sie des Weiteren eine oder mehrere Hilfsverbindungen umfasst, die aus schäumenden Tensiden und/oder Reinigungsmitteln, verdickenden und/oder gelierenden Tensiden, verdickenden und/oder gelierenden Mitteln, Stabilisierungsmitteln, filmbildenden Verbindungen, Lösungsmitteln und Co-Lösungsmitteln, hydrotropen Mitteln, plastifizierenden Mitteln, opazifizierenden Mitteln, Perlglanzmitteln, Sequestriermitteln, chelatbildenden Mitteln, Antioxydanzien, Parfüms, ätherischen Ölen, Konservierungsmitteln, Konditionierungsmitteln, desodorierenden Mitteln, Bleichmitteln, die zum Entfärben der Haare und der Haut bestimmt sind, Wirkstoffen, die dazu bestimmt sind, eine behandelnde und/oder schützende Wirkung gegenüber der Haut oder den Haaren mit sich zu bringen, Sonnenfiltern, mineralischen Füllstoffen oder Pigmenten, Partikeln, die einen visuellen Effekt vermitteln oder zur Einkapselung von Wirkstoffen bestimmt sind, Peeling-Partikeln, Texturmitteln, optischen Aufhellern, Abwehrmitteln für Insekten ausgewählt werden.

12. Verwendung der Zusammensetzung (C₁) nach einem der Ansprüche 1 bis 11 als topische kosmetische Zusammensetzung zur Säuberung und/oder zum Schutz und/oder zur Pflege der Haut und/oder zum Verbessern und/oder Bewahren des ästhetischen Aussehens der Haut, der Haare oder der Schleimhaut.

13. Verfahren zum Aufhellen einer Zusammensetzung, umfassend mindestens 90 Massen-% Wasser, wobei das Wasser in aufgelöster Form zwischen 0,02 Mol und 1 Mol je Liter von mindestens einem Salz (S) des Ammoniumkations oder eines einwertigen oder mehrwertigen Metallkations (S) mit einem organischen oder anorganischen Anion umfasst, und mindestens einen aus der Polymerisation stammenden vernetzten anionischen Polyelektrolyten (PA) in Gegenwart von mindestens einem Vernetzungsmittel, 2-Methyl-2-[(1-oxo-2-propenyl)amino]-1-propansulfonsäure, teilweise oder vollständig in Salzform vorliegend, mit mindestens einem neutralen Monomer, ausgewählt aus N,N-Dialkylacrylamiden, in denen jede der Alkylgruppen zwischen einem und vier Kohlenstoffatome und mindestens ein Monomer der Formel (I) beinhaltet: 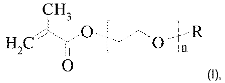 worin R einen linearen oder verzweigten Alkylrest repräsentiert, der von acht bis zwanzig Kohlenstoffatome beinhaltet und n eine Zahl größer als oder gleich eins und kleiner als oder gleich zwanzig repräsentiert,
wobei **das Verfahren** einen Schritt des Hinzufügens, in die Zusammensetzung, einer wirksamen Menge von mindestens einer Verbindung der Formel (II) **umfasst:** 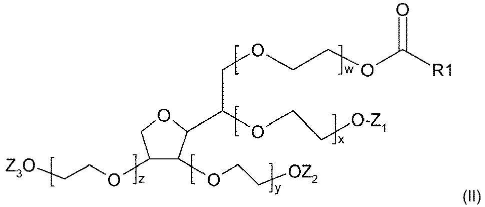 worin der Rest R₁-C(=O) einen gesättigten oder ungesättigten, linearen oder verzweigten, gegebenenfalls mit einer oder mehreren Hydroxylgruppen, die von sechzehn bis zweiundzwanzig Kohlenstoffatome beinhalten, substituierten Acylrest repräsentiert, Z₁, Z₂ und Z₃, identisch oder unterschiedlich, entweder das Wasserstoffatom oder den Rest R₁-C(=O) repräsentieren, x, y, z und w jeweils eine Zahl größer als oder gleich eins und kleiner als oder gleich vierzig repräsentieren, sodass die Summe (S_{M}) = x+y+z+w größer als oder gleich vier und kleiner als oder gleich vierzig ist.

## Claims

1. Composition (C₁) comprising per 100% of the weight thereof:
a) - Between 90 wt.-% and 99 wt.-% water comprising, in dissolved form, between 0.02 mol/L and 1 mol/L of at least one salt (S) of ammonium cation or of a monovalent or multivalent metal cation (S) with an organic or inorganic anion,
b) - Between 0.5 wt.-% and 5 wt.-% of at least one crosslinked anionic polyelectrolyte (PA) originating from polymerisation, in the presence of at least one crosslinking agent, partially or entirely salified 2-methyl 2-[(1-oxo 2-propenyl) amino] 1-propanesulfonic acid, with at least one neutral monomer chosen from N,N-dialkyl acrylamides, wherein each one of the alkyl groups comprises between one and four carbon atoms, and at least one monomer having formula (I): 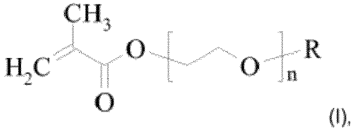 wherein R represents a linear or branched alkyl radical comprising from eight to twenty carbon atoms and n represents a number greater than or equal to one and less than or equal to twenty, and
c) - Between 0.5 wt.-% and 5 wt.-% of at least one compound having formula (II): 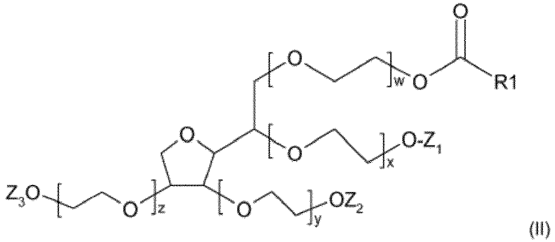 wherein the radical R₁-C(=O) represents a linear or branched, saturated or unsaturated acyl radical, optionally substituted with one or more hydroxyl groups, comprising from sixteen to twenty-two carbon atoms, Z₁, Z₂ and Z₃, identical or different, represent either the hydrogen atom, or the radical R₁-(C=O), x, y, z and w each represent a number greater than or equal to one and less than or equal to forty, such that the sum (S_{M}) = x+y+z+w is greater than or equal to four and less than or equal to forty,
it is understood that in said composition (C₁), the molar ratio (Rₐ) between said crosslinked anionic polyelectrolyte (PA) and said compound having formula (II) is greater than or equal to 0.2 and less than or equal to 2.0 and the molar ratio (R_{b}) between the compound having formula (II) and the salt (S) is greater than or equal to 0.02 and less than or equal to 1.

2. Composition (C₁) such as defined in claim 1, **characterised in that** said crosslinked anionic polyelectrolyte (PA) comprises, per 100 mol% of the constituting monomers thereof:
- From 20 mol% to 80 mol% of monomeric units originating from partially or entirely salified 2-methyl 2-[(1-oxo 2-propenyl) amino] 1-propanesulfonic acid
- From 15 mol% to 75 mol% of monomeric units originating from a neutral monomer chosen from N,N-dialkyl acrylamides, wherein each one of the alkyl groups comprises between one and four carbon atoms;
- From 0.5% to 5 mol% of monomeric units originating from a monomer having formula (I) such as defined above.

3. Composition (C₁) such as defined in claim 2, **characterised in that** said neutral monomer is N,N-dimethyl acrylamide.

4. Composition (C₁) such as defined in any one of claims 1 to 3, **characterised in that** said monomer having formula (I) is lauryl tetraethoxylated methacrylate.

5. Composition (C₁) such as defined in any one of claims 1 to 4, **characterised in that** in the formula (II), the radical R₁-C(=O) represents the radical cis-octadec-9-enoyl (or oleyl), Z₁, Z₂ and Z₃ are identical and represent the hydrogen atom, and the sum (S_{M}) = x+y+w+z is equal to 20.

6. Composition (C₁) such as defined in any one of claims 1 to 5, **characterised in that** said crosslinked anionic polyelectrolyte (PA) is a terpolymer of partially salified 2-methyl 2- [(1-oxo 2-propenyl) amino] 1-propanesulfonic acid in the form of ammonium salt, N,N-dimethyl acrylamide and lauryl tetraethoxylated methacrylate, crosslinked with trimethylol propanetriacrylate.

7. Composition (C₁) such as defined in any one of claims 1 to 6, **characterised in that** said crosslinked anionic polyelectrolyte (PA) comprise per 100 mol%:
- From 60 mol% to 80 mol% of monomeric units originating from partially salified 2-methyl 2-[(1-oxo 2-propenyl) amino] 1-propanesulfonic acid in the form of ammonium,
- From 15 mol% to 39.5 mol% of monomeric units originating from N,N-dimethyl acrylamide, and
- From 0.5 mol% to 5 mol% of monomeric units originating from lauryl tetraethoxylated methacrylate.

8. Composition (C₁) such as defined in any one of claims 1 to 7, **characterised in that** said salt (S) is chosen from halides, carbonates, bicarbonates, phosphates, nitrates, borates and ammonium sulphates or metal cation sulphates.

9. Composition (C₁) such as defined in any one of claims 1 to 8, **characterised in that** said salt (S) is a salt (S) of an ammonium cation or of a monovalent or multivalent metal cation (S) with an organic anion that has at least one carboxylate, sulphonate or sulphate function.

10. Composition (C₁) such as defined in any one of claims 1 to 9, **characterised in that** said salt (S) is chosen from sodium chloride, calcium chloride, magnesium chloride, calcium sulphate, ammonium sulphate, calcium carbonate, zinc sulphate, magnesium sulphate, sodium borate, sodium glycolate, sodium citrate, sodium salicylate, sodium lactate, sodium gluconate, zinc gluconate, manganese gluconate, copper gluconate and magnesium aspartate.

11. Composition (C₁) such as defined in any one of claims 1 to 10, **characterised in that** it further comprises, one or more auxiliary compounds chosen from foaming surfactants and/or detergents, thickening and/or gelling surfactants, thickening and/or gelling agents, stabilising agents, film-forming compounds, solvents and cosolvents, hydrotropic agents, plasticising agents, opacifying agents, pearlising agents, sequestrants, chelating agents, antioxidant agents, perfumes, essential oils, preservatives, conditioning agents, deodorants, bleaching agents intended for discolouring hairs and the skin, active ingredients intended to provide a treating and/or protective action with regards to the skin or hair, solar filters, mineral fillers or pigments, particles that procure a visual effect or intended for the encapsulation of active ingredients, exfoliating particles, texturing agents, optical brighteners, insect repellents.

12. Use of the composition (C₁) such as defined in any one or any of claims 1 to 11, as a topical cosmetic composition, for the cleaning and/or protection and/or care of the skin and/or to improve and/or to preserve the aesthetic appearance of the skin, hair or mucous membranes.

13. Method for lightening a composition comprising at least 90 wt.-% of water, said water comprising, in dissolved form between 0.02 mol/L and 1 mol/L of at least one salt (S) of an ammonium cation or of a monovalent or multivalent metal cation (S) with an organic or inorganic anion and at least one crosslinked anionic polyelectrolyte (PA) originating from polymerisation, in the presence of at least one crosslinking agent, partially or entirely salified 2-methyl 2-[(1-oxo 2-propenyl) amino] 1-propanesulfonic acid, with at least one neutral monomer chosen from N,N-dialkyl acrylamides, wherein each one of the alkyl groups comprises between one and four carbon atoms, and at least one monomer having formula (I): 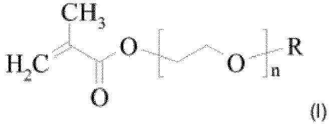 wherein R represents a linear or branched alkyl radical comprising from eight to twenty carbon atoms and n represents a number greater than or equal to one and less than or equal to twenty,
**said method comprising** a step of adding in said composition, of an effective quantity of at least one compound having formula (II): 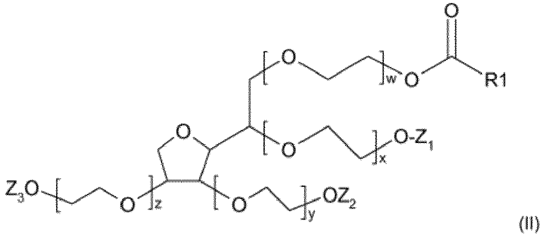 wherein the radical R₁-C(=O) represents a linear or branched, saturated or unsaturated acyl radical, optionally substituted with one or more hydroxyl groups, comprising from sixteen to twenty-two carbon atoms, Z₁, Z₂ and Z₃, identical or different, represent either the hydrogen atom, or the radical R₁-(C=O), x, y, z and w each represent a number greater than or equal to one and less than or equal to forty, such that the sum (S_{M}) = x+y+z+w is greater than or equal to four and less than or equal to forty.
